(19) Europäisches Patentamt
European Patent Office
Office européen des brevets

(11) Veröffentlichungsnummer: **0 020 301**
**A1**

(12) # EUROPÄISCHE PATENTANMELDUNG

(21) Anmeldenummer: **80810169.5**

(22) Anmeldetag: **22.05.80**

(51) Int. Cl.³: **C 07 C 91/30,** A 61 K 31/135 // C07C65/26, C07C65/36, C07C103/29, C07C103/38, C07C45/46, C07C49/84, C07C43/23, C07C41/26, C07C43/225, C07C41/22, C07C93/14, C07C29/10, C07C29/62

(30) Priorität: **28.05.79 CH 4944/79**

(43) Veröffentlichungstag der Anmeldung: **10.12.80 Patentblatt 80/25**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Fuhrer, Walter, Dr., Munzackerweg 11, CH-4402 Frenkendorf (CH)**
Erfinder: **Kühnis, Hans, Dr., Lange Gasse 26, CH-4052 Basel (CH)**

(54) **Neue basisch substituierte Phenole, Verfahren zu deren Herstellung, pharmazeutische Präparate enthaltend diese neuen Verbindungen sowie deren Verwendung.**

(57) Die Erfindung betrifft neue basisch substituierte Phenole der ormel

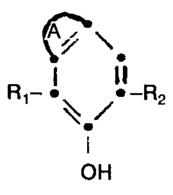

(I)

worin einer der Reste $R_1$ und $R_2$ jeweils für Halogen und der andere für die Gruppe der Formel $-CH_2NH_2$ steht, und A Niederalkylen mit 3 bis 6 Kohlenstoffatomen bedeutet, wovon 3 bis 4 Kohlenstoffatome in der unverzweigten Kette stehen, sowie Salze, insbesondere pharmazeutisch verwendbare, nicht-toxische Säureadditionssalze solcher Verbindungen, Verfahren zu deren Herstellung, pharmazeutische Präparate, die solche Verbindungen enthalten, sowie deren Verwendung. Die neuen Verbindungen können als Diuretika und Saluretika z.B. zur Behandlung von Oedemen, außerdem aber auch der Hypertonie verwendet werden.

EP 0 020 301 A1

0020301

CIBA-GEIGY AG
Basel (Schweiz)

4-12372/+

BEZEICHNUNG GEÄNDERT
siehe Titelseite

Basisch substituierte Phenole

Die Erfindung betrifft neue, basisch substituierte Phenole, ferner Verfahren zu deren Herstellung, pharmazeutische Präparate, die diese neuen Verbindungen enthalten, sowie ihre Verwendung.

Die Erfindung betrifft insbesondere neue basisch substituierte Phenole der Formel

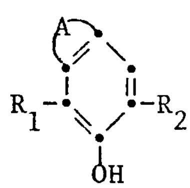

(I)

worin einer der Reste $R_1$ und $R_2$ jeweils für Halogen und der andere für die Gruppe der Formel $-CH_2NH_2$ steht, und A Niederalkylen mit 3 bis 6 Kohlenstoffatomen bedeutet, wovon 3 bis 4 Kohlenstoffatome in der unverzweigten Kette stehen, sowie Salze, insbesondere pharmazeutisch verwendbare, nicht-toxische Säureadditionssalze solcher Verbindungen.

Halogen steht für Fluor, Chlor, Brom oder Jod. Niederalkylen ist z.B. 1,3-Propylen, 1,1-Dimethyl-1,3-propylen, 1,1-Dimethyl-1,4-butylen oder 1,4-Butylen, kann aber auch verzweigt sein und ist dann z.B. 2,4-Butylen oder 3,3-Dimethyl-2,4-butylen.

Salze von Verbindungen der Formel I sind in erster Linie Säureadditionssalze, und insbesondere pharmazeutisch verwendbare, nicht-toxische Säureadditionssalze mit geeigneten anorganischen Säuren, wie Chlorwasserstoffsäure, Bromwasserstoffsäure, Schwefelsäure oder Phosphorsäure, oder mit geeigneten organischen aliphatischen, cycloaliphatischen, aromatischen, araliphatischen oder heterocyclischen Carbon- oder Sulfonsäuren, wie Ameisensäure, Essigsäure, Propionsäure, Bernsteinsäure, Glycolsäure, Milchsäure, Aepfelsäure, Weinsäure, Zitronensäure, Ascorbinsäure, Maleinsäure, Fumarsäure, Brenztraubensäure, Benzoesäure, Anthranilsäure, 4-Hydroxybenzoesäure, Salicylsäure, Phenylessigsäure, Embonsäure, Methansulfonsäure, Aethansulfonsäure, Hydroxyäthansulfonsäure, Aethylensulfonsäure, 4-Chlorbenzolsulfonsäure, Toluolsulfonsäure, Naphthalinsulfonsäure, Sulfanilsäure oder Cyclohexylaminsulfonsäure.

Infolge der engen Beziehungen zwischen den neuen Verbindungen in freier Form und in Form ihrer Salze sind unter den freien Verbindungen und unter den Salzen sinn- und zweckgemäss gegebenenfalls auch die entsprechenden Salze bzw. freien Verbindungen zu verstehen.

Die neuen Verbindungen besitzen wertvolle pharmakologische Eigenschaften, die insbesondere in einer starken diuretischen sowie natriuretischen und chloruretischen Wirksamkeit bestehen. Die Kaliumausscheidung wird weniger stark als die Natriumausscheidung erhöht. Diese Effekte können nach peroraler Verabreichung solcher

Verbindungen in Form einer Suspension in Tragacanthschleim mittels Schlundsonde in Dosen von 10 bis 100 mg/kg an Ratten und ab 0,1 mg/kg an Hunden beobachtet werden. Die Wirkungen an der Ratte können durch die Menge des während drei Stunden nach Verabreichung der Substanz ausgeschiedenen Urins im Vergleich zu einer unbehandelten Kontrollgruppe bestimmt werden, während die Wirksamkeit am Hund durch die während 5 Stunden nach Verabreichung ausgeschiedene, in Abständen von jeweils einer Stunde gesammelte Menge Urin im Vergleich zu der während einer Stunde vor Substanzgabe ausgeschiedenen Menge ermittelt werden kann.

Aufgrund dieser Eigenschaften können die neuen Verbindungen als Diuretika und Saluretika z.B. zur Behandlung von Oedemen, ausserdem aber auch der Hypertonie verwendet werden.

Die Erfindung betrifft insbesondere Verbindungen der Formel I, worin einer der Reste $R_1$ und $R_2$ jeweils für Halogen und der andere für die Gruppe der Formel $-CH_2NH_2$ steht, und A Niederalkylen mit 3 bis 5 Kohlenstoffatomen bedeutet, worin 3 Kohlenstoffatome in der unverzweigten Kette stehen, oder deren Salze, insbesondere deren pharmazeutisch verwendbaren nicht-toxischen Säureadditionssalze.

Die Erfindung betrifft vor allem Verbindungen der Formel I, worin einer der Reste $R_1$ und $R_2$ jeweils für Halogen und der andere für die Gruppe der Formel $-CH_2NH_2$ steht, und A für 1,1-Dimethyl-1,3-propylen oder 3,3-Dimethyl-2,4-butylen steht, oder deren Salze, insbesondere deren pharmazeutisch verwendbaren nicht-toxischen Säureadditionssalze.

Insbesondere betrifft die Erfindung die in den Beispielen beschriebenen Verbindungen der Formel I, oder deren Salze, insbesondere deren pharmazeutisch verwendbaren, nicht-toxischen Säureadditonssalze.

Die neuen Verbindungen können nach an sich bekannten Methoden hergestellt werden.

So kann man diese erhalten, indem man z.B. in einer Verbindung der Formel

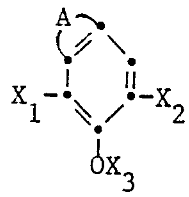

(II),

worin einer der Reste $X_1$ und $X_2$ jeweils Halogen, und der andere, die Gruppe der Formel $-CH_2NH_2$ Ia oder einen in die Gruppe der Formel Ia überführbaren Rest darstellen, $X_3$ für Wasserstoff steht, oder $X_1$ und $X_3$ zusammen bzw. $X_2$ und $X_3$ zusammen einen unter Bildung der Hydroxygruppe und der Gruppe Ia abspaltbaren Rest darstellen und A die unter der Formel I angegebene Bedeutung hat, mit der Massgabe, dass eines der Symbole $X_1$ und $X_2$ für einen in die Gruppe der Formel Ia überführbaren Rest steht, und das andere Halogen bedeutet, oder in einem Salz davon, einen in eine Gruppe der Formel Ia überführbaren Rest $X_1$ oder $X_2$ in die Gruppe der Formel Ia überführt oder die Reste $X_1$ und $OX_3$ bzw. $X_2$ und $OX_3$ gleichzeitig in die Gruppe Ia bzw. die Hydroxygruppe umwandelt, und, wenn erwünscht, eine erhaltene freie Verbindung in ein Salz oder ein erhaltenes Salz in eine freie Verbindung überführt, oder ein erhaltenes Racemat in die optischen Antipoden auftrennt.

In der Form von Salzen verwendbare Ausgangsstoffe werden in erster Linie in der Form von Säureadditionssalzen, insbesondere von entsprechenden Salzen mit anorganischen Säuren, z.B. Mineralsäuren, sowie von organischen Säuren verwendet.

Ein in die Gruppe der Formel Ia überführbarer Rest ist z.B. ein mittels Reduktion einschliesslich Hydrogenolyse, oder durch Solvolyse einschliesslich Hydrolyse, Acidolyse oder Alkoholyse in die Gruppe der Formel Ia überführbarer Rest.

Ein mittels Reduktion einschliesslich Hydrogenolyse in die Gruppe der Formel Ia überführbarer Rest ist z. B. eine Gruppe der Formel $-CH_2-NHX_4$ (IIa) oder der Formel $-(C=Y)-NHX_4'$ (IIb), worin Y den Oxo- oder Thioxorest darstellt, und $X_4'$ Wasserstoff oder einen unter den Bedingungen der Reduktion abspaltbaren und durch Wasserstoff ersetzbaren Rest bedeutet, und $X_4$ einen unter den Bedingungen der Reduktion abspaltbaren und durch Wasserstoff ersetzbaren Rest darstellt.

Eine hydrogenolytisch abspaltbare Gruppe $X_4$ oder $X_4'$ ist in erster Linie eine $\alpha$-Arylniederalkylgruppe, wie eine gegebenenfalls substituierte 1-Phenylniederalkylgruppe, worin Niederalkyl bis zu 7 Kohlenstoffatome hat und worin Substituenten z. B. Niederalkyl oder Niederalkoxy mit jeweils bis zu 7 Kohlenstoffatomen z. B. Methyl oder Methoxy, sein können, und ganz besonders Benzyl. Eine durch $X_3$ und $X_4$ oder $X_4'$ bzw. dem in der nachfolgenden Formel IIc definierten $X_5$ zusammen gebildete hydrogenolytisch abspaltbare Gruppe ist, z.B. gegebenenfalls substituiertes 1-Phenylniederalkyliden, wobei Niederalkyliden bis zu 7 Kohlenstoffatome hat, und worin Substituenten, insbesondere des Phenylteils, z.B. Niederalkyl, wie Methyl, oder Niederalkoxy, wie Methoxy, sein können, und insbesondere Benzyliden.

Insbesondere geeignet zur Reduktion von Verbindungen der Formel II mit einer Gruppe der Formel IIb sind Hydridreduktionsmittel, wie z.B. Diboran, Natriumborhydrid, Natriumcyanoborhydrid oder Lithiumaluminiumhydrid. Für die Hydrogenolyse von Verbindungen der Formel II mit einer Gruppe der Formeln IIa oder IIb, worin $X_4'$ verschieden von Wasserstoff ist, insbesondere geeignet ist katalytisch aktivierter Wasserstoff, wie Wasserstoff in Gegenwart eines geeigneten Hydrierkatalysators, z.B. eines Nickel-, Platin- oder Palladiumkatalysators.

Gruppierungen der Formel IIb, worin Y jeweils eine Thioxogruppe bedeutet, werden durch reduktive Entschwefelung, z.B. durch Behandeln mit einem Hydrierkatalysator, wie Raney-Nickel, in die Gruppierung der Formel Ia umgewandelt. Durch Beschränkung der Menge Reduktionsmittel bzw. des eingesetzten Katalysators und geeignete Wahl der Reduktionsbedingungen ist dafür zu sorgen, dass aromatisch gebundenes Halogen nicht abgespalten wird.

Weitere mittels Reduktion einschliesslich Hydrogenolyse in die Gruppe der Formel Ia überführbare Reste sind solche der Formeln $-CH{=}NX_5$ (IIc), $-CH{=}NOH$ (IId) oder $-C{\equiv}N$ (IIe), worin $X_5$ einen hydrogenolytisch abspaltbaren durch Wasserstoff ersetzbaren Rest, z.B. wie oben für $X_4$ angegeben, bedeutet. Die Reduktion von Verbindungen der Formel II mit den Resten der Formeln IIc einschliesslich der Abspaltung der Gruppe $X_5$, oder mit den Resten der Formeln IId oder IIe kann z.B. durch Behandeln mit katalytisch aktiviertem Wasserstoff, wie Wasserstoff in Gegenwart eines geeigneten Hydrierkatalysators z.B. eines Nickel-, Platin- oder Palladium-, aber auch eines Rhodium- oder Rutheniumkatalysators erfolgen. Oder man arbeitet mit einem geeigneten Hydridreduktionsmittel, wie Diboran oder einem Alkalimetallborhydrid, z.B. Natriumborhydrid oder Natriumcyanoborhydrid. Die Reduktion eines Ausgangsstoffes der Formel II mit der Gruppe der Formel IId kann ausserdem mittels eines Alkalimetalls, z.B. Natrium, in einem Niederalkanol, etwa Aethanol, durchgeführt werden.

Weitere mittels Reduktion in die Gruppe der Formel Ia überführbare Reste sind Reste der Formeln $-CH_2N_3$ (IIf) oder $-CH_2NO_2$ (IIg), von denen die Gruppe der Formel IIf mittels katalytisch aktiviertem Wasserstoff, wie Wasserstoff in Gegenwart eines Hydrierkatalysators z.B. wie oben beschrieben, oder

mittels eines Leichtmetallhydrids, z.B. Lithiumaluminiumhydrid, zur Gruppe der Formel Ia reduziert wird, während die Gruppe der Formel $II_g$ z.B. mittels katalytisch aktiviertem Wasserstoff, etwa wie oben beschrieben, oder mit nascierendem Wasserstoff, z.B. mittels eines geeigneten Metalls, wie Eisen oder gegebenenfalls amalgamiertem Zink in einer Säure, z.B. einer Mineralsäure, wie wässeriger Salzsäure, zur Gruppe der Formel Ia reduziert wird. Die Reduktion der Gruppe der Formel IIf kann auch mittels eines Leichtmetallhydrids, etwa Lithiumaluminiumhydrid, erfolgen.

Unter reduktiv abspaltbaren Resten $X_4$ bzw. $X_4'$ und/oder $X_5$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl, wie 2,2,2-Trichloräthoxycarbonyl, die z.B. beim Behandeln mit einem reduzierenden Schwermetall, wie Zink, oder mit einem reduzierenden Schwermetallsalz, wie einem Chrom(II)-salz, z.B. -chlorid oder -acetat, üblicherweise in Gegenwart einer organischen Carbonsäure, wie Ameisensäure oder Essigsäure, und von Wasser abgespalten werden können.

Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° bis etwa +150° , und/oder in einem geschlossenen Gefäss unter Druck und/oder in einer Inertgas-, z.B. Stickstoffatmosphäre, durchgeführt.

Ein mittels Solvolyse wie Hydrolyse, Acidolyse oder Alkoholyse in die Gruppe der Formel I überführbarer Rest $X_1$ oder $X_2$ ist eine Gruppe der Formel $-CH_2NX_6X'_6$ (IIh), worin $X_6$ Wasserstoff und $X'_6$, insbesondere einen Acylrest, wie den Acylrest einer organischen Carbonsäure, z.B. Niederalkanoyl, wie Acetyl, Halogenniederalkanoyl, wie Halogenacetyl, z.B. Choracetyl, oder Carbamoyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebenen Bedeutungen haben, und in erster Linie Trityl darstellt, oder $X_6$ und $X'_6$ zusammen einen zweibasischen Acylrest, z.B. Phthaloyl bedeuten.
Mittels Solvolyse, wie Hydrolyse, Acidolyse oder Alkoholyse, in die Gruppe Ia unter gleichzeitiger Bildung der Hydroxygruppe überführbare Reste sind durch $X_3$ und $X'_6$ zusammen gebildete Reste, z.B. hydrolytisch abspaltbare Gruppen, wie Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden oder Cycloalkyliden, z.B. Cyclopentyliden oder Cyclohexyliden.

Hydrolytisch abspaltbare Gruppen $X_6'$, wie Acylreste von organischen Carbonsäuren, z.B. Niederalkanoyl oder Halogenniederalkanoyl, Carbamoyl, oder Halbester der Kohlensäure, z.B. Niederalkoxycarbonyl, ferner z.B. Tritylreste, sowie durch die Reste $X_3$ und $X_6'$ zusammen gebildete Niederalkyliden-, 1-Phenyl-niederalkyliden- oder Cycloalkylidengruppen, oder durch die Reste $X_6$ und $X_6'$ zusammen gebildete zweibasiche Acylreste, wie Phthaloyl oder die Carbonylgruppe können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxides oder -carbonats oder in Gegenwart von Ammoniak oder eines Amins, wie Isopropylamin, oder Hydrazinhydrat, abgespalten werden.

Acidolytisch abspaltbare Reste $X_6'$ sind insbesondere gewisse Acylreste von Halbestern der Kohlensäure, wie z.B. tert.- Niederalkoxycarbonyl oder gegebenenfalls substituierte Diphenylmethoxycarbonylreste, ferner auch tert.-Niederalkylreste, z.B. tert.-Butyl; diese können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig in Gegenwart eines aktivierenden Mittels, wie Anisol), sowie mit Ameisensäure abgespalten werden.

Die obigen Reaktionen werden in üblicher Weise und in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel II können, sofern sie neu sind, nach an sich bekannten Methoden, gegebenenfalls in situ, hergestellt werden.

So kann man z.B. ein Ausgangsmaterial der Formel II mit der Gruppe IIa oder IIb als $X_1$ oder $X_2$ erhalten, indem man eine Verbindung der Formel

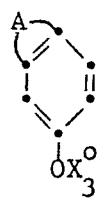

(III),

worin $X_3^o$ z.B. mittels saure Mittel, etwa konz. Mineralsäure, wie konz. Bromwasserstoffsäure, oder Aluminiumchlorid abspaltbares Niederalkyl, z.B. Methyl ist, mit einem reaktionsfähigen Derivat der Essigsäure, z.B. Acetylchlorid analog den Bedingungen nach Friedel-Crafts, z.B. in Gegenwart von Zinntetrachlorid zu einer Verbindung der Formel

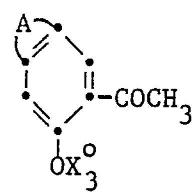

(IV),

umsetzt, diese Verbindung mittels eines geeigneten Oxidationsmittels, etwa Natriumhypochloritlösung, zur Verbindung der Formel

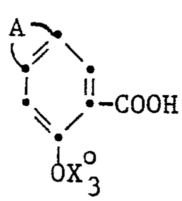

(V)

oxidiert, anschliessend diese Verbindung auf übliche Weise, z.B. über das mittels Thionylchlorid erhaltene Säurechlorid und dessen Umsetzung mit einem Amin der Formel $H_2N-X_4$ oder $H_2N-X_4'$ in die die Carbamoylgruppe enthaltende Verbindung der Formel

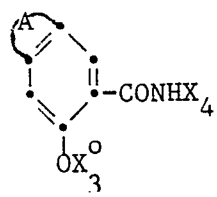

(VI),

bzw. der Formel

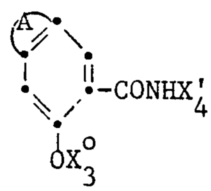

(VIa),

überführt, anschliessend zur Herstellung eines Ausgangsmaterials der Formel II mit der Gruppe IIa eine Verbindung der Formel VI durch Reduktion, z.B. mittels eines Hydridreduktionsmittels, wie etwa Lithiumaluminiumhydrid, in eine die Gruppe IIa enthaltende Verbindung der Formel

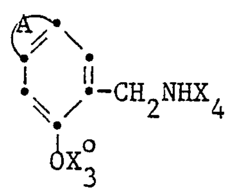

(VIb)

umwandelt, anschliessend in einer Verbindung der Formel VI, VIa oder VIb die Gruppe $-OX_3^o$ in die Hydroxygruppe, z.B. mittels saurer Mittel, wie z.B. Aluminiumchlorid oder einer konzentrierten Mineralsäure, wie konzentrierter Bromwasserstoffsäure überführt, die erhaltene die freie Hydroxygruppe aufweisende Verbindung VII anschliessend zu einem Ausgangsmaterial der Formel II halogeniert, wobei zur Einführung von Chlor z.B. elementares Chlor oder Sulfurylchlorid, von Brom oder Jod z.B. elementares oder in Form eines reaktionsfähigen Derivats, z.B. Brommonochlorid oder Jodmonochlorid, vorliegendes Brom oder Jod, oder letzteres als Lösung in Kaliumjodid verwendet werden kann.

Ein Ausgangsmaterial der Formel II mit einer Gruppe IIe als $X_1$ oder $X_2$ ist durch Umwandlung der Gruppe $-CONHX_4'$, worin $X_4'$ für Wasserstoff steht, in einer Verbindung der Formel VI oder VII in die Gruppe $-C{\equiv}N$ und die sich anschliessende, z.B. analog wie beschrieben, durchzuführende Reaktionsfolge zugänglich. Die Umwandlung wird durch Abspaltung von Wasser auf übliche Weise, z.B. mittels wasserentziehender Mittel, wie Thionylchlorid oder Phosphorpentachlorid, oder einem Carbodiimid, z.B. Cyclohexylcarbodiimid vorgenommen. Ein Ausgangsmaterial der Formel II mit der Gruppe IIc oder IId ist durch Umsetzung eines entsprechenden Aldehyds oder dessen reaktionsfähigen Derivaten wie einem Acetal, etwa dem Diäthylacetal, der Formel

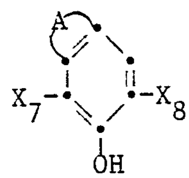 (VIII),

worin einer der Reste $X_7$ oder $X_8$ für die Gruppe -CHO steht und der andere Halogen bedeutet, mit einer Verbindung der Formel $H_2N-X_5$ oder mit Hydroxylamin zugänglich, wobei solche Ausgangsstoffe auch <u>in situ</u> hergestellt werden können. Eine Verbindung der Formel (VIII) wiederum kann durch Einführen der Gruppe -CHO in ein entsprechend substituiertes Halogenphenol, z.B. mittels Chloroform in alkalischem Medium oder durch Umsetzung mit Hexamethylentetramin in Gegenwart von Borsäure oder einem Aethoxyäthanol-Borsäure-Glyceringemisch erhalten werden. Diese Umsetzungen werden auf übliche Weise vorgenommen.

Ein Ausgangsmaterial der Formel II mit der Gruppe IIf oder IIg kann durch Umsetzung einer Verbindung der Formel

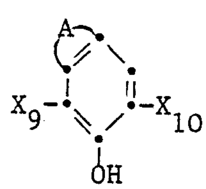 (IX),

worin einer der Reste $X_9$ und $X_{10}$ Halogen bedeutet und der andere für die Gruppe der Formel $-CH_2X_{11}$ (X), steht, worin $X_{11}$ für eine reaktionsfähige veresterte Hydroxygruppe, z.B. eine Methansulfonyloxygruppe steht, oder Halogen, insbesondere Chlor, Brom oder Jod bedeutet, mit einem Salz der Stickstoffwasserstoffsäure, insbesondere einem Metallsalz, etwa Natrium- oder Kaliumazid, bzw. einem Metallsalz der salpetrigen Säure, z.B. Silbernitrit oder Kaliumnitrit, erhalten werden. Die Umsetzungen erfolgen in üblicher Weise; z.B. erfolgt die Umsetzung mit Natrium- oder Kaliumazid in einem polaren Lösungsmittel, etwa einem Niederalkanol, z.B. Aethanol, oder einem gegebenenfalls N-niederalkylierten Fettsäureamid, wie z.B. Dimethylformamid oder N-Methylacetamid. Die Umsetzung, z.B. mit Silbernitrit erfolgt auf bekannte Weise in einem Halogen-, z.B. einem Chlorkohlenwasserstoff, wie Methylenchlorid, während man z.B. Kaliumnitrit in einem polaren Lösungsmittel, z.B. Acetonitril, vorzugsweise in Gegenwart eines Katalysators, etwa eines Kronenäthers wie etwa 18-Krone-6, oder Natriumnitrit in einem niederen Fettsäureamid, z.B. Dimethylformamid, zur Umsetzung mit einer Verbindung der Formel (IX) bringt. Diese Umsetzungen werden in an sich bekannter Weise vorgenommen.

Ein Ausgangsmaterial der Formel IX wiederum wird erhalten, indem man in einer Verbindung der Formel

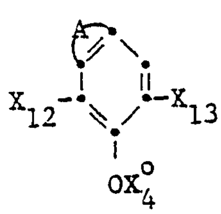

(XI)

worin $X_4^o$ eine z.B. mittels saurer Mittel, wie Aluminiumchlorid, abspaltbare und durch Wasserstoff ersetzbare Gruppe, wie Niederalkyl, z.B. Methyl, oder eine Acylgruppe, wie die Acetylgruppe bedeutet, und einer der Reste $X_{12}$ oder $X_{13}$ Halogen ist und der andere für Wasserstoff steht, die Gruppe der Formel $-CH_2X_{11}$ (X) einführt. Diese Reaktion kann man durch Umsetzung der Verbindung der Formel (XI) mit Formaldehyd in alkalischem Medium, z.B. in wässriger Natriumhydroxid - oder Natriumbicarbonatlösung und Umsetzung der erhaltenen Hydroxymethylverbindung z.B. mit Methansulfonylchlorid in Pyridin zur entsprechenden Methansulfonyloxyverbindung bewirken. Oder man setzt eine Verbindung der Formel (XI) mit Formaldehyd und einem Halogenwasserstoff, insbesondere Chlor- oder Bromwasserstoff in Gegenwart eines Kondensationsmittels, z.B. Zinkchlorid, zur entsprechenden Halogenmethyl-Verbindung um und wandelt anschliessend die Gruppe der Formel $-OX_4^o$ in die Hydroxygruppe, z.B. mittels Hydrolyse in Gegenwart saurer Mittel, etwa wie oben beschrieben, um. Anstelle von Formaldehyd kann auch ein reaktionsfähiges Derivat davon, z.B. ein Acetal, wie etwa Formaldehyddimethylacetal, eingesetzt werden. Schliesslich kann die Einführung z.B. einer Chlormethylgruppe auch mittels Chlormethyläther erfolgen.

Ein Ausgangsstoff der Formel II mit einer Gruppe IIh als $X_1$ oder $X_2$ kann erhalten werden, indem man z.B. eine Verbindung der Formel

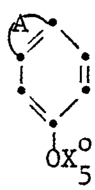

(XII),

worin $X_5^o$ für Wasserstoff steht, oder eine, z.B. mittels saurer Mittel, etwa wie vorhin beschrieben, abspaltbare und durch Wasserstoff ersetzbare Gruppe, z.B. einer der vorhin definierten Acylreste, wie Acetyl, oder Niederalkyl, wie Methyl, darstellt, halogeniert und die erhaltene Verbindung mit einer Verbindung der Formel $HOCH_2-NX_6X_6'$ (XIIa) umsetzt.

Diese Reaktion wird in üblicher Weise in saurem Medium, z.B. in einer Niederalkancarbonsäure, etwa Essigsäure oder einem Gemisch derselben mit einer Mineralsäure, wie konzentrierter Schwefelsäure, vorgenommen. In einer so erhaltenen Verbindung wird eine gegebenenfalls vorhandene Schutzgruppe $X_5^o$ z.B. mittels saurer Mittel, etwa wie vorhin beschrieben, abgespalten, und durch Wasserstoff ersetzt.

Diese Umsetzungen werden in an sich bekannter Weise vorgenommen.

Die neuen Verbindungen der Formel I können ebenfalls erhalten werden, indem man in einer Verbindung der Formel

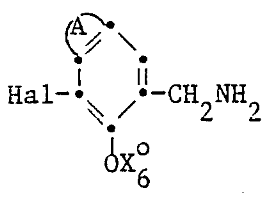

(XIII)

worin $X_6^o$ einen abspaltbaren, durch Wasserstoff ersetzbaren Rest bedeutet, oder in einem Salz davon, den Rest $X_6^o$ abspaltet und durch Wasserstoff ersetzt und gewünschtenfalls die zusätzlichen Verfahrensschritte durchführt.

Ein abspaltbarer und durch Wasserstoff ersetzbarer Rest $X_6^o$ ist z.B. ein mittels Reduktion einschliesslich Hydrogenolyse, oder durch Solvolyse, einschliesslich Hydrolyse, Acidolyse oder Alkoholyse abspaltbarer Rest.

Ein hydrogenolytisch abspaltbarer Rest $X_6^o$ ist in erster Linie eine $\alpha$-Arylniederalkylgruppe, wie eine gegebenenfalls substituierte 1-Phenylniederalkylgruppe, worin Niederalkyl bis zu 7 Kohlenstoffatome hat und worin Substituenten z.B. Niederalkyl oder Niederalkoxy mit jeweils bis zu 7 Kohlenstoffatomen, z.B. Methyl oder Methoxy, sein können, und ganz besonders Benzyl.

Die reduktive Abspaltung des Restes $X_6^o$ kann insbesondere z.B. durch Behandeln mit katalytisch aktiviertem Wasserstoff, wie Wasserstoff in Gegenwart eines geeigneten Hydrierkatalysators z.B. eines Nickel-, Platin- oder Palladium-, aber auch eines Rhodium- oder Rutheniumkatalysators erfolgen. Oder man arbeitet mit einem geeigneten Hydridreduktionsmittel, wie Diboran oder einem Alkalimetallborhydrid, z.B. Natriumborhydrid oder Natriumcyanoborhydrid.

Unter reduktiv abspaltbaren Resten $X_6^o$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl, wie 2,2,2-Trichloräthoxycarbonyl, die z.B. beim Behandeln mit einem reduzierenden Schwermetall, wie Zink, oder mit einem reduzierenden Schwermetallsalz, wie einem Chrom(II)-salz, z.B. -chlorid oder -acetat, üblicherweise in Gegenwart einer organischen Carbonsäure, wie Ameisensäure oder Essigsäure, und von Wasser abespalten werden können.

Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° bis etwa +150°, und/oder in einem geschlossenen Gefäss unter Druck und/oder in einer Inertgas-, z.B. Stickstoffatmosphäre, durchgeführt.

Ein mittels Solvolyse wie Hydrolyse, Acidolyse oder Alkoholyse abspaltbarer und durch Wasserstoff ersetzbarer Rest $X_6^o$ ist z.B. ebenso wie eine, wie oben beschrieben, solvolytisch wie hydrolytisch, acidolytisch oder alkoholytisch spaltbare und durch Wasserstoff ersetzbare Gruppe $X_6'$ insbesondere ein Acylrest, wie der Acylrest einer organischen Carbonsäure, z.B. Niederalkanoyl, wie Acetyl, Halogenniederalkanoyl, wie Halogenacetyl, z.B. Chloracetyl, oder Carbamoyl, oder Aroyl, wie Benzoyl, ferner der Acylrest eines Halbesters der Kohlensäure, wie Niederalkoxycarbonyl, z.B. Methoxycarbonyl, Aethoxycarbonyl oder tert.-Butyloxycarbonyl , 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebenen Bedeutungen haben, und in erster Linie Trityl darstellt. Eine auf diese Weise abspaltbare Gruppe $X_6^o$ kann ausserdem Niederalkyl, z.B. Methyl, oder eine 1-Phenylniederalkylgruppe, wie gegebenenfalls, z.B. wie oben definiert, substituiertes Benzyl sein.

Hydrolytisch abspaltbare Reste $X_6^o$, wie Acylreste von organischen Carbonsäuren, z.B. Niederalkanoyl oder Halogenniederalkanoyl, Carbamoyl, oder Halbester der Kohlensäure, z.B. Niederalkoxycarbonyl, ferner z.B. Tritylreste können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxides oder -carbonats oder in Gegenwart von Ammoniak oder eines Amins, wie Isopropylamin abgespalten werden.

Acidolytisch abspaltbare Reste $X_6^o$ sind insbesondere gewisse Acylreste von Halbestern der Kohlensäure, wie z.B. tert.- Niederalkoxycarbonyl oder gegebenenfalls substituierte Diphenylmethoxycarbonylreste, ferner auch tert.-Niederalkylreste, z.B. tert.-Butyl; diese können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig in Gegenwart eines aktivierenden Mittels, wie Anisol), sowie mit Ameisensäure abgespalten werden.

Die obigen Reaktionen werden in üblicher Weise und in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel XIII können, sofern sie neu sind, nach an sich bekannten Methoden, gegebenenfalls in situ, hergestellt werden.

So kann man z.B. eine Verbindung der Formel

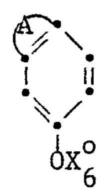

(XIV),

in üblicher Weise z.B. wie oben beschrieben, z.B. mit elementarem Chlor, Brom oder Jod oder mittels eines Dihalogenids, wie Bromchlorid oder Jodchlorid, zu einer Verbindung der Formel

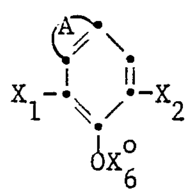

(XV),

worin einer der Reste $X_1$ oder $X_2$ Halogen bedeutet und der andere Wasserstoff ist, halogenieren, anschliessend in diese Verbindung, z.B. analog wie oben beschrieben, etwa mittels Formaldehyd im alkalischem

Medium und anschliessender Veresterung mit einer Säure, etwa einer Mineralsäure wie konzentrierter Salzsäure oder konzentrierter Bromwasserstoffsäure, oder mit einer Sulfonsäure, etwa Methansulfonsäure, vorzugsweise in Form eines reaktionsfähigen Derivats, z.B. Methansulfonylchlorid, in Pyridin, oder mittels Formaldehyd und konzentrierter Salzsäure in Gegenwart eines Kondensationsmittels, etwa Zinkchlorid, die oben erläuterte Gruppe der Formel X einführen, und in der erhaltenen Verbindung der Formel

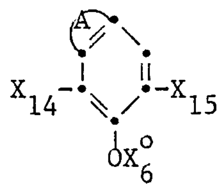

(XVI),

worin einer der Reste $X_{14}$ und $X_{15}$ Halogen und der andere die Gruppe der Formel X bedeutet, worin $X_{11}$ für eine reaktionsfähige veresterte Hydroxygruppe, z.B. Halogen, wie Chlor oder Brom, oder eine Sulfonyloxygruppe, z.B. Methansulfonyloxy steht, die Gruppe $X_{11}$ gegen die primäre Aminogruppe in üblicher Weise, austauscht. Dieses geschieht z.B. durch Umsetzung mit Hexamethylentetramin und Zersetzung der erhaltenen Verbindung mit einer verdünnten Säure, z.B. einer verdünnten Mineralsäure, wie verdünnter Salzsäure, oder durch Umsetzung mit einer Phthalimidverbindung, z.B. Phthalimidhalium, und anschliessender Abspaltung des Phthalylrestes mittels Hydrolyse, z.B. wässriger Mineralsäuren, oder mittels Hydrazinhydrat.

Die neuen Verbindungen der Formel I können ebenfalls erhalten werden, indem man eine Verbindung der Formel

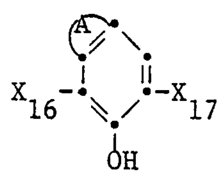

(XVII),

worin einer der Reste $X_{16}$ und $X_{17}$ die Gruppe der Formel Ia und der andere Wasserstoff bedeutet, halogeniert, und, wenn, erwünscht, die

zusätzlichen Verfahrensschritte durchführt. So kann man z.B. für die Einführung von Chlor, Brom oder Jod diese in elementarer Form, letzteres auch in Form einer Lösung von Jod in Kaliumjodid, verwenden, oder man setzt für die Einführung von Chlor oder Brom Sulfurylchlorid oder Sulfurylbromid ein. Für die Einführung von Halogen kann auch ein reaktionsfähiges Derivat davon, z.B. Brommonochlorid oder Jodmonochlorid, verwendet werden.
Zweckmässigerweise werden diese Umsetzungen in einem Lösungsmittel, etwa einem Halogenkohlenwasserstoff wie Chloroform oder Tetrachlorkohlenstoff, oder in saurem Medium, z.B. in einer Niederalkancarbonsäure, z.B. Essigsäure, vorgenommen, falls elementares Chlor oder Brom verwendet wird, während die Einführung von Brom oder Jod unter Verwendung eines reaktionsfähigen Derivats davon, z.B. in einer verdünnten Mineralsäure, wie wässriger Chlorwasserstoff- oder Bromwasserstofflösung vorgenommen werden kann. Diese Umsetzungen werden in an sich bekannter Weise durchgeführt.

Zur Herstellung eines Ausgangsmaterials der Formel XVII kann man z.B. so vorgehen, dass man in einer Verbindung der Formel

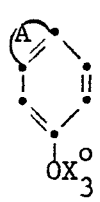

(XVIII),

worin $X_3^o$ eine z.B. mittels saurer Mittel, etwa wie oben beschrieben, abspaltbare und durch Wasserstoff ersetzbare Gruppe bedeutet, die Gruppe der Formel X, z.B. wie oben beschrieben, einführt, und die z.B. Chlor oder Brom darstellende Gruppe $X_{11}$ gegen die primäre Aminogruppe auf übliche Weise, z.B. durch Umsetzen mit Hexamethylentetramin in einem organischen Lösungsmittel, wie Chloroform, und Spalten des erhaltenen Addukts mit wässeriger Mineralsäure, z.B. verdünnter Salzsäure, oder durch Umsetzen mit Phthalimidkalium und Hydrolysieren der N-Phthalimido-Verbindung mit einer Säure, z.B. konzentrierter Salzsäure, oder mittels einer Base wie z.B. Hydrazinhydrat, austauscht.

Die neuen Verbindungen der Formel I können ebenfalls erhalten werden, indem man in einer Verbindung der Formel

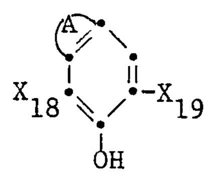

( XIX),

worin einer der Reste $X_{18}$ und $X_{19}$ Halogen und der andere die Gruppe der Formel $-CH_2X_{20}$ (XX) bedeutet, worin $X_{20}$ für eine reaktionsfähige veresterte Hydroxygruppe steht, die Gruppe $X_{20}$ gegen die primäre Aminogruppe austauscht, und, wenn erwünscht, die zusätzlichen Verfahrensschritte durchführt.

Eine reaktionsfähige veresterte Hydroxygruppe $X_{20}$ ist eine, durch eine starke Säure, insbesondere eine starke anorganische Säure wie eine Halogenwasserstoffsäure, insbesondere Chlor-, Brom oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die Umwandlung der Gruppe $X_{20}$ in die primäre Aminogruppe kann z.B. durch Umsetzung mit einer die primäre Aminogruppe liefernden Verbindung, z.B. mit Hexamethylentetramin bewerkstelligt

werden. Dieses wird z.B. mit einer Verbindung der Formel XIX in einem organischen Lösungsmittel, z.B. einem Halogenkohlenwasserstoff, wie Chloroform, zum entsprechenden Addukt umgesetzt, welches anschliessend in saurem Medium, z.B. mittels wässeriger Mineralsäure, etwa verdünnter Salzsäure, in eine Verbindung der Formel I umgewandelt wird. Man kann ferner eine Verbindung der Formel XIX z.B. mit Phthalimidkalium in einem geeigneten Lösungsmittel, z.B. einem solchen polaren Charakters, wie einem Niederalkanol, z.B. Aethanol, oder einem gegebenenfalls N-alkylierten Fettsäureamid, z.B. Dimethylformamid, oder N-Methylacetamid, zur entsprechenden N-Phthalimidoverbindung umsetzen, aus welcher dann, z.B. wie oben beschrieben, mittels Hydrolyse, etwa mit einer Mineralsäure, wie konz. Salzsäure, oder mittels einer Base, wie z.B. Hydrazinhydrat die Verbindung der Formel I erhalten wird. Diese Umsetzungen werden in üblicher Weise vorgenommen.

Ein Ausgangsstoff der Formel XIX kann in an sich bekannter Weise erhalten werden, indem man in einer Verbindung der Formel

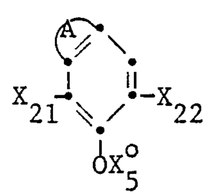

(XXI),

worin einer der Reste $X_{21}$ und $X_{22}$ für Halogen steht und der andere Wasserstoff bedeutet und $X_5^o$ Wasserstoff ist oder eine abspaltbare durch Wasserstoff ersetzbare Gruppe, z.B. eine Niederalkylgruppe wie Methyl, oder eine Acylgruppe, wie eine Niederalkanoylgruppe z.B. Acetyl, darstellt, die Gruppe der oben erläuterten Formel XX einführt, und einen gegebenenfalls vorhandenen Rest $X_5^o$ z.B. mittels saurer Mittel wie Aluminiumchlorid, oder mittels einer Säure, wie einer Mineralsäure z.B. Schwefelsäure, abspaltet und durch Wasserstoff ersetzt. Die Einführung der Gruppe der Formel XX kann, z.B. wie

oben beschrieben, mittels Formaldehyd in alkalischem Medium und anschliessender Umwandlung der Hydroxygruppe in eine der Gruppe $X_{20}$ entsprechende reaktionsfähige veresterte Hydroxygruppe in üblicher Weise, z.B. wie oben beschrieben, erfolgen. Oder man setzt eine Verbindung der Formel (XXI), worin die Hydroxygruppe vorzugsweise, etwa wie angegeben, geschützt ist, mit Formaldehyd und Halogenwasserstoff, etwa Chlor- oder Bromwasserstoff in Gegenwart eines Kondensationsmittels, wie Zinkchlorid, zur entsprechenden Halogenmethylverbindung um und überführt eine gegebenenfalls vorhandene, z.B. wie angegeben, geschützte Hydroxygruppe in die freie Hydroxygruppe. Die Einführung einer der Formel XX entsprechenden Chlormethylgruppe kann auch mittels Chlormethyläther erfolgen. Diese Umsetzungen werden in an sich bekannter Weise vorgenommen.

Die neuen Verbindungen der Formel I können ferner erhalten werden, indem man in einer Verbindung der Formel

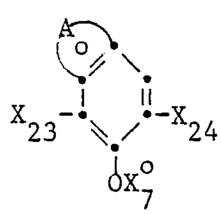

(XXII),

worin $A_o$ einen Niederalkenylenrest bedeutet, oder die Gruppe der Formel -A'-C(=O)-(Ib) darstellt, worin A' für den eine Methylengruppe weniger aufweisenden Rest A steht, einer der Reste $X_{23}$ und $X_{24}$ jeweils Halogen und der andere die Gruppe der Formel Ia oder eine mittels Reduktion in die Gruppe der Formel Ia überführbaren Rest darstellen, und $X_7^o$ Wasserstoff oder einen mittels Hydrogenolyse abspaltbaren und durch Wasserstoff ersetzbaren Rest, z.B. wie oben beschrieben, bedeutet, oder in einem Salz davon, den Rest $A_o$ zur Gruppe A reduziert, und zugleich eine gegebenenfalls vorhandene, mittels Reduktion in die Gruppe der Formel Ia überführbare Gruppe $X_{23}$ oder $X_{24}$ zur Gruppe der Formel Ia reduziert, und/oder zugleich einen gegebenenfalls vorhandenen, mittels Hydrogenolyse abspaltbaren und durch Wasserstoff ersetzbaren Rest $X_7^o$

abspaltet und durch Wasserstoff ersetzt, und wenn erwünscht, die zusätzlichen Verfahrensschritte durchführt. Niederalkenylen ist z.B. 1-Propen-1,3-ylen, 2-Propen-1,3-ylen, 1-Buten-1,4-ylen, oder 2-Buten-1,4-ylen, welches aber auch verzweigt sein kann und z.B. 1-Propen-2,3-ylen oder 1-Methyl-2-buten-2,4-ylen darstellt. In einer Gruppe der Formel Ib kann die Carbonylgruppe in jeder der möglichen Stellungen stehen. Demnach entspricht eine Gruppe der Formel Ib z.B. den Formeln $-CO-CH_2-CH_2-$ (XXIII), $-CH_2-CO-CH_2-$ (XXIV), $-CH_2-CO-CH_2-CH_2-$ (XXV), $-CH_2-CH_2-CH_2-CO-$ (XXVI), $-CO-C(CH_3)_2-CH_2-$ (XXVII) oder $-CH_2-C(CH_3)_2-CH_2-CO-$ (XXVIII).

Mittels Reduktion in die Gruppe der Formel Ia überführbare Gruppen sind z.B. solche der Formeln IIa, IIb, IIc, IId, IIe, IIf und IIg wie vorhin beschrieben.

Die Reduktion von Niederalkenylenresten $A_o$ wird in üblicher Weise, z.B. mittels katalytisch aktiviertem Wasserstoff in Gegenwart eines Hydrierkatalysators, z.B. eines Nickel-, Platin- oder Palladiumkatalysators durchgeführt, während die Reduktion einer Gruppe der Formel Ib mit einem geeigneten Hydridreduktionsmittel, wie Diboran, oder einem Alkalimetallborhydrid z.B. Natriumcyanoborhydrid erfolgt. Gegebenenfalls vorhandene reduzierbare Gruppen IIa, IIb, IIc, IId, IIe, IIf oder IIg werden mittels dieser Reduktionsmethoden gleichzeitig zur Gruppe der Formel Ia reduziert. Diese Reduktionsreaktionen werden in an sich bekannter Weise, üblicherweise in Gegenwart eines inerten Lösungsmittels, und wenn notwendig, unter Kühlen oder Erwärmen z.B. in einem Temperaturbereich von etwa -20° bis etwa +150° und/oder in einem geschlossenen Gefäss unter Druck und/oder in einer Inertgas-, z.B. Stickstoffatmosphäre durchgeführt.

Ein Ausgangsstoff der Formel XXII kann in üblicher Weise erhalten werden, indem man z.B. in einer Verbindung der Formel

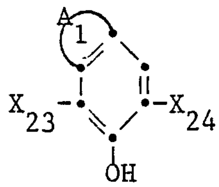 (XXIX),

worin $A_1$ einem mono-halogenierten, z.B. monochlorierten, Niederalkylenrest A entspricht, eine Gruppe $A_1$ durch Abspaltung von Halogen-, z.B. Chlorwasserstoff, z.B. mittels einer starken Base, wie wässrigem Kaliumhydroxid, in eine Niederalkenylengruppe $A_o$ überführt.

Ein Ausgangsstoff der Formel XXII, worin $A_o$ z.B. für die Gruppe XXIII oder XXVI steht, kann erhalten werden, indem man in einem, wie oben beschriebenen, Ausgangsmaterial der Formel V die Gruppe A zur Gruppe der Formel -A'-(C=O)- (Ib), worin A' die eine Methylengruppe weniger aufweisende Gruppe A darstellt, in üblicher Weise z.B. mittel Chromtrioxid oxidiert, anschliessend die erhaltene Verbindung in an sich bekannter Weise, etwa über das mittels Thionylchlorid erhaltene Säurechlorid und dessen anschliessender Umsetzung z.B. mit einem Amin der Formel $H_2N-X_4$ in die Verbindung der Formel

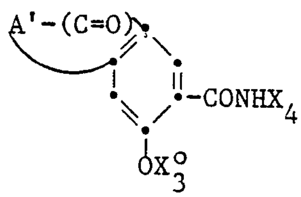 (XXX),

umwandelt, in dieser Verbindung wie oben unter Formel VI beschrieben, die Gruppe $-OX_3^o$ in die Hydroxygruppe umwandelt und die erhaltene Verbindung anschliessend, wie beschrieben, halogeniert.

Ein Ausgangsstoff der Formel XXII, worin $A_o$ z.B. für die Gruppe XXIII oder XXVI steht, kann ferner z.B. aus einer Carbonsäure der Formel

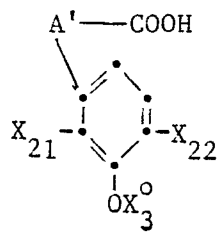

(XXXI),

oder einem reaktionsfähigen Derivat davon, z.B. einem Halogenid wie dem Chlorid, unter den Bedingungen nach Friedel-Crafts mittels Aluminiumchlorid in einem inerten Lösungsmittel, z.B. Benzol und nachfolgender Umwandlung der Gruppe $-OX_3^o$ in die Hydroxygruppe z.B. wie beschrieben, erhalten werden. Diese Umsetzungen werden in üblicher Weise vorgenommen.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden-, carbonaten oder hydrogencarbonaten oder Ionenaustauschern, in die freien Verbindungen übergeführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren, nicht toxischen Salzen eignen.

Diese und andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate vorliegen. Die Ausgangsstoffe können auch als optische Antipoden eingesetzt werden.

Erhaltene Racematgemische können auf Grund der physikalischchemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedener Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, OO'-Di-(p-Toluolyl-Weinsäure, Aepfelsäure, Mandelsäure, Camphersulfonsäure, Glutaminsäure, Asparaginsäure oder Chinasäure.

Vorteilhafterweise isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z.B. analog wie in dem Beispiel, beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z.B. oralen oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder Calciumstearat, und/oder Polyäthylenglykol, enthalten. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen

sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten, Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50% Lyophilisate bis zu 100% des Aktivstoffes.

Die Dosierung kann von verschiedenen Faktoren, wie Applikationsweise, Spezies, Alter und/oder individuellem Zustand abhängen. So liegen die täglich zu verabreichenden Dosen bei oraler Applikation an Warmblütern von etwa 70 kg vorzugsweise zwischen etwa 0,05 und 2,0 g.

Die folgenden Beispiele dienen zur Illustration der Erfindung; Temperaturen werden in Celsiusgraden angegeben.

<u>Beispiel 1</u>: Zu 144 ml einer 1-m. eisgekühlten Diboranlösung in Tetrahydrofuran gibt man während 10 Minuten 7,2 g 4-Jod-5-hydroxy-6-carbamoyl-1-indanon portionenweise zu und lässt die klare Lösung während 2 Tagen bei Raumtemperatur stehen. Unter Rühren fügt man dann 200 ml Essigsäureäthylester tropfenweise zu, rührt noch während 30 Minuten bei Raumtemperatur und entfernt die Lösungsmittel am Rotationsverdampfer. Den Rückstand nimmt man in 100 ml 5-n. methanolischer Salzsäure auf und erhitzt das Gemisch während 2 Stunden unter Rückfluss. Nach Entfernen des Lösungsmittels am Rotationsverdampfer nimmt man den Rückstand in Methanol auf, filtriert die heisse Lösung über Tierkohle, engt das Filtrat auf ein kleines Volumen ein und fügt Essigsäureäthylester bis zur beginnenden Kristallisation zu. Die Kristalle werden abgesaugt und aus Methanol/Essigsäureäthylester umkristallisiert, wonach man des 4-Jod-6-(aminomethyl)-5-indanol als Hydrochlorid vom Smp. 207-208° erhält.

Das als Ausgangsmaterial verwendete 4-Jod-5-hydroxy-6-carbamoyl-1-indanon kann wie folgt hergestellt werden:

a) Eine Lösung von 78 g 5-Methoxyindan und 37,4 ml Acetylchlorid in 400 ml Benzol wird unter Eiskühlung bei höchstens 30° Innentemperatur mit 61,7 ml Zinntetrachlorid versetzt. Die erhaltene Emulsion wird anschliessend über Nacht bei Raumtemperatur gerührt, dann auf Eis gegossen und das erhaltene Gemisch mit Aether extrahiert. Die organische Phase wird abgetrennt, mit verdünnter Salzsäure, dann mit verdünnter Natronlauge extrahiert, über Natriumsulfat getrocknet und eingedampft, wonach man das 5-Methoxy-6-acetyl-indan als schwach gelbes Oel erhält.

b) 166 g Chlorgas werden in 1600 ml einer 3,5-n. Natronlauge eingeleitet, die Lösung anschliessend auf 55° erwärmt und mit 82 g 5-Methoxy-6-acetyl-indan versetzt. Durch Eiskühlung wird die Temperatur bei 65-70°, und nach Abklingen der exothermen Reaktion noch 1 Stunde bei 65° gehalten. Anschliessend wird das Reaktionsgemisch auf Raumtemperatur abgekühlt, mit einer Lösung von 49,2 g Natriumbisulfit in 190 ml Wasser versetzt und mehrmals mit Methylenchlorid extrahiert. Die wässerige Phase wird mittels eines Filterhilfsmittels klar filtriert und durch Zugabe von 191 ml konz. Salzsäure neutralisiert, wobei die 5-Methoxy-indan-6-carbonsäure in Form schwach gelber Kristalle vom Smp. 107-108° ausfällt.

c) Eine Suspension von 96,1 g 5-Methoxy-indan-6-carbonsäure in einem Gemisch von 250 ml Eisessig und 144 ml Acetylchlorid wird während einer Stunde bei einer Temperatur von höchstens 30° mit einer Mischung von 70 g Chromtrioxid, 36 ml Wasser und 280 ml Eisessig versetzt, wonach eine klare Lösung entsteht, die später zu kristallisieren beginnt. Nach 2-stündigem Rühren bei Raumtemperatur giesst man das Reaktionsgemisch auf 2 Liter Eiswasser, filtriert den ausgefallenen Festkörper ab, wäscht diesen mit Wasser und trocknet im Hochvakuum bei 70°, wonach man das 5-Methoxy-6-carboxy-1-indanon vom Smp. 232-238° erhält.

d) Eine durch Eis gekühlte Suspension von 20,6 g 5-Methoxy-6-carboxy-1-indanon in 80 ml Dimethylformamid wird im Verlaufe von 10 Minuten mit 9 ml Thionylchlorid versetzt. Nach 10 Minuten Stehen bei Raumtemperatur bildet sich eine braun-grüne Lösung, welche in ein Gemisch von 200 ml konz. Ammoniak und Eis eingerührt wird. Der gebildete braune Niederschlag wird abgesaugt, mit Wasser, dann mit Aceton und schliesslich mit Aether gewaschen und im Hochvakuum bei 80° getrocknet, wonach man rohes 5-Methoxy-6-carbamoyl-1-indanon erhält, welches als solches weiterverarbeitet wird.

e) Eine Suspension von 19,2 g 5-Methoxy-6-carbamoyl-1-indanon in 250 ml Aethylenchlorid wird mit 39 g wasserfreiem Aluminiumchlorid versetzt und das Gemisch während 2 Stunden unter Stickstoff unter Rückfluss erhitzt. Nach Entfernen des Lösungsmittels am Rotationsverdampfer wird der Rückstand mit Eis und 100 ml konz. Salzsäure versetzt. Das ungelöste Produkt wird abfiltriert, in 200 ml 1-n. Natronlauge gelöst, die dunkle Lösung über Aktivkohle filtriert und anschliessend in der Wärme durch Zugabe von Salzsäure sauer eingestellt. Der ausgefallene Festkörper wird abgesaugt, mit viel Wasser gewaschen und bei 120° getrocknet, wonach man das 5-Hydroxy-6-carbamoyl-1-indanon als beigefarbenes Pulver erhält; Smp. 300° (unter Zersetzung).

f) 5,3 g 5-Hydroxy-6-carbamoyl-1-indanon werden in 29,2 ml 1-n. Natronlauge gelöst und die Lösung mit 29 ml Wasser verdünnt. Hierzu fügt man unter Rühren 29,2 ml einer 1-m. Lösung von Jod in einer 1-m. wässerigen Kaliumjodid-Lösung, wonach sich bald eine hellbraune Suspension bildet. Nach weiterem Rühren während 25 Minuten bei Raumtemperatur gibt man verdünnte Salzsäure zu, filtriert durch einen Hartfilter und wäscht das Produkt mit viel Wasser nach. Nach dem Trocknen im Hochvakuum bei 80° erhält man das 4-Jod-5-hydroxy-6-carbamoyl-1-indanon als schwach beigefarbenes Pulver.

Beispiel 2: Eine Lösung von 10,3 g 1,1-Dimethyl-6-[(N-chloracetyl)-aminomethyl]-4-jod-5-indanol in einem Gemisch von 120 ml Aethanol und 25 ml konz. Salzsäure wird während 5 Stunden unter Rückfluss gerührt. Die orangefarbene Lösung wird anschliessend am Rotationsverdampfer vom Lösungsmittel befreit und der Rückstand noch jeweils 3 mal mit Isopropanol versetzt und zur Entfernung restlicher Salzsäure jedesmal zur Trockne verdampft. Durch mehrmaliges Umkristallisieren aus Isopropanol/Diäthyläther erhält man das 1,1-Dimethyl-6-aminomethyl-4-jod-5-indanol-hydrochlorid als farbloses Salz vom Smp. 172-173°.

Beispiel 3: Analog der im Beispiel 2 beschriebenen Verfahrensweise erhält man aus 1,1-Dimethyl-6-jod-4-[(N-chloracetyl)-aminomethyl]-5-indanol das 1,1-Dimethyl-6-jod-4-aminomethyl-5-indanol-hydrochlorid-hemihydrat vom Smp. 152-153°.

Die als Ausgangsmaterialien verwendeten Substanzen 1,1-Dimethyl-4-[(N-chloracetyl)-aminomethyl]-6-jod-5-indanol bzw. 1,1-Dimethyl-4-jod-6-[(N-chloroacetyl)-aminomethyl]-5-indanol können wie folgt hergestellt werden:

a) Eine Lösung von 10,0 g m-Methoxydihydrozimtsäuremethylester (Beilstein, 2. Erg. Werk, Band 10; S. 145) in 180 ml absolutem Diäthyläther wird unter trockenem Stickstoff bei 0-2° gerührt. Im Verlaufe von 30 Minuten werden 54,1 ml einer 2-m.Lösung von Methyllithium in Diäthyläther zugetropft und die sich bildende Suspension anschliessend während 2 Stunden bei Raumtemperatur gerührt. Man giesst das Reaktionsgemisch auf eine Mischung von Eis und 1-n.Salzsäure, extrahiert 2 mal mit Aether, trocknet die Aetherphase über Natriumsulfat, entfernt das Lösungsmittel am Rotationsverdampfer und chromatographiert das zurückbleibende Oel mit einem Gemisch von Essigsäureäthylester/Hexan 1:4 an Kieselgel, wobei man das 4-(2-Methoxyphenyl)-2-methyl-2-butanol als Oel erhält.

b) Eine auf 0° abgekühlte Lösung von 8,15 g 4-(2-Methoxyphenyl)-2-methyl-2-butanol in 160 ml Methylenchlorid wird portionenweise mit 5,62 g wasserfreiem Aluminiumchlorid versetzt. Die resultierende rosarote Suspension wird während 3 Stunden bei Raumtemperatur gerührt, dann auf ein Gemisch von Eis und 1-n.Salzsäure gegossen und die Wasserphase mit Methylenchlorid extrahiert. Die organische Phase wird abgetrennt, über Natriumsulfat getrocknet und vom Lösungsmittel befreit. Das als Oel zurückbleibende 1,1-Dimethyl-5-methoxy-indan wird zusammen mit 6,9 g D,L-Methionin in 43 ml Methansulfonsäure gelöst und die Lösung unter Stickstoff während 6 Stunden bei 100° gerührt. Die

abgekühlte, dunkelrote Reaktionslösung wird auf Eis gegossen und mit konz. Ammoniak auf pH 9 eingestellt. Nach Extraktion mit Essigsäureäthylester und Entfernung des Lösungsmittels am Rotationsverdampfer wird der Rückstand mit Essigsäureäthylester/Hexan 1:10 als Laufmittel an Kieselgel chromatographiert, wobei man das 1,1-Dimethyl-5-indanol als kristallisierendes Oel erhält.

c) Eine Lösung von 14,6 g 1,1-Dimethyl-5-indanol in 100 ml Eisessig wird mit 17,5 g Jodmonochlorid versetzt und während 3 1/2 Stunden unter Rückfluss erhitzt. Das dunkle Reaktionsgemisch wird dann auf Eis gegossen, und die Wasserphase 2 mal mit Essigsäureäthylester extrahiert. Die vereinigten organischen Phasen werden 2 mal mit 0,1-n.Natriumthiosulfatlösung und 1 mal mit gesättigter wässriger Natriumchloridlösung extrahiert, über Natriumsulfat getrocknet und am Rotationsverdampfer vom Lösungsmittel befreit. Zur Abtrennung vom Ausgangsmaterial wird der Rückstand an Kieselgel mit Methylenchlorid/Cyclohexan 1:2 chromatographiert, wobei das Isomerengemisch bestehend aus 1,1-Dimethyl-4-jod-5-indanol und 1,1-Dimethyl-6-jod-5-indanol im Verhältnis von etwa 2:1 als Oel anfällt.

d) 14,7 g des erhaltenen, aus 1,1-Dimethyl-4-jod-5-indanol und 1,1-Dimethyl-6-jod-5-indanol im Verhältnis von etwa 2:1 bestehenden Isomerengemisches werden in einem Gemisch von 100 ml Eisessig und 10 ml konz.Schwefelsäure gelöst. Nach Zugabe von 9,6 g N-Hydroxymethylchloracetamid wird während 12 Stunden bei Raumtemperatur gerührt, anschliessend das Reaktionsgemisch auf Eiswasser gegossen und die Suspension 2 mal mit 100 ml Essigsäureäthylester extrahiert. Die vereinigten organischen Phasen werden über Natriumsulfat getrocknet, vom Lösungsmittel befreit und der Rückstand an Kieselgel mit Essigsäureäthylester/Hexan 1:4 chromatographiert, wobei zuerst das 1,1-Dimethyl-4-jod-6-[(N-chloracetyl)-aminomethyl]-5-indanol und dann

das 1,1-Dimethyl-6-jod-4-[(N-chloracetyl)-aminomethyl]-5-indanol eluiert werden; beide Verbdinungen stellen kristallisierende Oele dar.

Beispiel 4: 4,8 g des rohen aus 1,1-Dimethyl-4-aminomethyl-5-indanol-hydrochlorid und 1,1-Dimethyl-6-aminomethyl-5-indanol-hydrochlorid bestehenden Isomerengemisches werden in 400 ml warmem Wasser gelöst. Bei einer Innentemperatur von 50° wird eine Lösung von 4,1 g Jodmonochlorid in 20 ml 3-n.Salzsäure in einem Guss zugegeben. Das Reaktionsgemisch wird anschliessend 3 Stunden bei dieser Temperatur gerührt, abgekühlt, mit konz. Ammoniak auf pH 8 eingestellt und mit Methylenchlorid mehrmals extrahiert. Die vereinigten organischen Phasen werden über Natriumsulfat getrocknet, das Lösungsmittel am Rotationsverdampfer entfernt und der Rückstand zur Abtrennung von Verunreinigungen und zur Trennung der Isomeren an Kieselgel mit einem Gemisch von Chloroform/Methanol/Ammoniak im Verhältnis 350:50:1 chromatographiert, wobei zuerst das 1,1-Dimethyl-4-aminomethyl-6-jod-5-indanol und nachfolgend das 1,1-Dimethyl-4-jod-6-aminomethyl-5-indanol eluiert wird.

Zur Herstellung des Hydrochlorids wird jeweils die freie Base in Isopropanol gelöst und die Lösung mit überschüssiger methanolischer Salzsäure versetzt. Nach Entfernung des Lösungsmittels am Rotationsverdampfer wird der jeweilige Rückstand je zweimal aus Isopropanol-Diäthyläther umkristallisiert, wobei man das 1,1-Dimethyl-6-aminomethyl-4-jod-5-indanol-hydrochlorid vom Smp. 172-173° und das 1,1-Dimethyl-6-jod-4-aminomethyl-5-indanol-hydrochlorid-hemihydrat vom Smp. 152-153° erhält.

Die Ausgangsstoffe können wie folgt hergestellt werden:

a) Die Lösung von 8,1 g des nach Beispiel 3b) erhaltenen 1,1-Dimethyl-5-indanols in einem Gemisch von 100 ml Eisessig und 10 ml konz.Schwefelsäure wird nach Zugabe von 9,3 g N-Hydroxymethyl-

chloracetamid während 12 Stunden bei Raumtemperatur gerührt, das Reaktionsgemisch anschliessend auf Eiswasser gegossen und die sich bildende Suspension 2 mal mit Essigsäureäthylester extrahiert. Die vereinigten organischen Phasen werden über Natriumsulfat getrocknet, vom Lösungsmittel befreit und der Rückstand, der ein Isomerengemisch von 1,1-Dimethyl-6-[(N-chloracetyl)-aminomethyl]-5-indanol und 1,1-Dimethyl-4-[(N-chloracetyl)-aminomethyl]-5-indanol darstellt, als solcher in der nächsten Stufe eingesetzt.

b) Eine Lösung von 7,0 g des erhaltenen Isomerengemisches in einem Gemisch von 50 ml Aethanol und 16 ml konz. Salzsäure wird während 5 Stunden unter Rückfluss gerührt. Die Reaktionslösung wird anschliessend am Rotationsverdampfer vom Lösungsmittel befreit und der Rückstand mehrmals mit Aethanol versetzt und die Lösung jedesmal zur Trockne verdampft. Das zurückbleibende rohe Isomerengemisch, bestehend aus 1,1-Dimethyl-4-aminomethyl-5-indanol-hydrochlorid und 1,1-Dimethyl-6-aminomethyl-5-indanol-hydrochlorid wird als solches weiterverarbeitet.

<u>Beispiel 5:</u> 4,3 g rohes, aus 1,1-Dimethyl-4-aminomethyl-5-methoxyindan-hydrochlorid und 1,1-Dimethyl-6-aminomethyl-5-methoxyindan-hydrochlorid bestehendes Isomerengemisch werden in einem Gemisch von 50 ml Essigsäure und 50 ml 48%iger Bromwasserstoffsäure 2 Stunden unter Rückfluss erhitzt. Das Lösungsmittel wird am Rotationsverdampfer weitgehend entfernt, der Rückstand in verdünntem Ammoniak aufgenommen und mit Methylenchlorid mehrmals extrahiert.

Die Trennung der Isomeren und die Herstellung der Hydrochloride erfolgt analog Beispiel 4, wobei das 1,1-Dimethyl-6-aminomethyl-4-jod-5-indanol-hydrochlorid vom Smp. 172-173° und das 1,1-Dimethyl-6-jod-4-aminomethyl-5-indanol-hydrochlorid-hemihydrat vom Smp. 152-153° erhalten wird.

Die Ausgangsstoffe können wie folgt hergestellt werden:

a) Eine Lösung von 6,3 g 1,1-Dimethyl-5-methoxy-indan in 50 ml Eisessig wird mit 6,7 g Jodmonochlorid versetzt und während 3 Stunden unter Rückfluss erhitzt. Aufarbeitung analog Beispiel 3c) ergibt das Isomerengemisch von 1,1-Dimethyl-4-jod-5-methoxy-indan und 1,1-Dimethyl-6-jod-5-methoxy-indan, welches als solches in der nächsten Stufe eingesetzt wird.

b) Eine Lösung von 5,3 g rohem Isomerengemisch, bestehend aus 1,1-Dimethyl-4-jod-5-methoxyindan und 1,1-Dimethyl-6-jod-5-methoxyindan in einem Gemisch von 40 ml Essigsäure und 4 ml konz.Schwefelsäure wird portionenweise mit 3,2 g N-Hydroxymethylchloracetamid versetzt und das Reaktionsgemisch während 24 Stunden bei Raumtemperatur gerührt. Anschliessend giesst man auf Eiswasser, extrahiert mehrmals mit Essigsäureäthylester und filtriert die vereinigten organischen Phasen zur Abtrennung von Verunreinigungen durch Kieselgel. Das nach dem Abdampfen des Lösungsmittels resultierende rohe Isomerengemisch, bestehend aus 1,1-Dimethyl-4-jod-6-[(N-chloracetyl)-aminomethyl]-5-methoxyindan und 1,1-Dimethyl-6-jod-4-[(N-chloracetyl)-aminomethyl]-5-methoxyindan wird als solches in der nächsten Stufe eingesetzt.

c) 3,8 g des erhaltenen rohen Isomerengemisches werden in einer Mischung von 50 ml Aethanol und 10 ml konz.Salzsäure während 5 Stunden unter Rückfluss gerührt. Das Reaktionsgemisch wird anschliessend am Rotationsverdampfer vom Lösungsmittel befreit und der Rückstand, bestehend aus rohem Isomerengemisch von 1,1-Dimethyl-4-jod-6-aminomethyl-5-methoxy-indan-hydrochlorid und 1,1-Dimethyl-4-aminomethyl-6-jod-5-methoxy-indan-hydrochlorid, als solcher weiterverarbeitet.

Beispiel 6: In ein Bombenrohr werden bei -40° 50 ml flüssiges Ammoniak eingefüllt. Unter magnetischem Rühren wird bei dieser Temperatur 3,3 g eines rohen Gemisches von 1,1-Dimethyl-4-jod-6-chlormethyl-5-indanol

und 1,1-Dimethyl-6-jod-4-chlormethyl-5-indanol, gelöst in 5 ml Dioxan, langsam zugetropft. Das Bombenrohr wird verschlossen und unter weiterem Rühren langsam auf Raumtemperatur gebracht. Nach 24 Stunden Stehen bei Raumtemperatur wird erneut abgekühlt, das überschüssige Ammoniak abgedampft und der Rückstand an Kieselgel mit einem Gemisch von Chloroform/Methanol/Ammoniak im Verhältnis 350:50:1 chromatographiert, wobei die beiden Isomeren 1,1-Dimethyl-6-aminomethyl-4-jod-5-indanol und 1,1-Dimethyl-6-jod-4-aminomethyl-5-indanol getrennt werden.

Die Herstellung der Hydrochloride erfolgt analog Beispiel 4, wobei das 1,1-Dimethyl-6-aminomethyl-4-jod-5-indanol-hydrochlorid vom Smp. 172-173° und das 1,1-Dimethyl-6-jod-4-aminomethyl-5-indanol-hydrochlorid-hemihydrat vom Smp. 152-153° erhalten werden.

Das als Ausgangsmaterial benötigte Isomerengemisch von 1,1-Dimethyl-4-jod-6-chlormethyl-5-indanol und 1,1-Dimethyl-6-jod-4-chlormethyl-5-indanol kann wie folgt erhalten werden.

Eine Lösung von 5,6 g des nach Beispiel 3c) erhaltenen Isomerengemisches, bestehend aus 1,1-Dimethyl-4-jod-5-indanol und 1,1-Dimethyl-6-jod-5-indanol in 60 ml konz.Salzsäure und 0,5 ml konz.Schwefelsäure wird mit 4,4 g Methylal versetzt. Anschliessend leitet man unter Rühren und bei einer Temperatur von 70° Chlorwasserstoffgas während 4 Stunden ein. Nach Abkühlen wird das Reaktionsgemisch auf Eiswasser gegossen und mit Methylenchlorid extrahiert. Das nach Entfernung des Lösungsmittels erhaltene rohe Isomerengemisch, bestehend aus 1,1-Dimethyl-4-jod-6-chlormethyl-5-indanol und 1,1-Dimethyl-6-jod-4-chlormethyl-5-indanol wird sofort weiterverarbeitet.

<u>Beispiel 7:</u> Eine Lösung von 18 g rohem Isomerengemisch bestehend aus 4-Jod-6-[(N-chloracetyl)-aminomethyl]-5-indanol und 6-Jod-4-[(N-chloracetyl)-aminomethyl]-5-indanol in einem Gemisch von 300 ml Alkohol und 30 ml konzentrierter Salzsäure wird während 18 Stunden unter Rückfluss gerührt. Die dunkle Lösung wird am Rotationsverdampfer vom Lösungsmittel befreit, der Rückstand in Wasser aufgenommen und mit Ammoniaklösung alkalisch gestellt. Das dabei ausfallende Produkt wird abfiltriert und auf einer Kieselgelsäule mit einem Lösungsmittelgemisch aus Chloroform/Methanol/Ammoniak im Verhältnis 350:50:1 chromatographiert, wobei zuerst das 4-Aminomethyl-6-jod-5-indanol und anschliessend das 4-Jod-6-aminomethyl-5-indanol eluiert wird.

Mit methanolischer Salzsäure werden die beiden Basen in die Hydrochloride überführt und anschliessend aus Isopropanol umkristallisiert, wobei man das 4-Aminomethyl-6-jod-5-indanol-hydrochlorid vom Smp. 200-210° (Zersetzung) und das 4-Jod-6-aminomethyl-5-indanol-hydrochlorid vom Smp 207-208° erhält.

Das als Ausgangsmaterial verwendete Isomerengemisch von 4-Jod-6-[(N-chloracetyl)-aminomethyl]-5-indanol und 6-Jod-4-[(N-chloracetyl)-aminomethyl]-5-indanol kann z.B. wie folgt erhalten werden:

a) Eine Gemisch von 33,5 g 5-Indanol, 42 g Natriumbicarbonat, 400 ml Wasser und 400 ml Methanol wird bei Raumtemperatur gerührt. Im Verlaufe einer Stunde wird eine Lösung von 31,8 g Jod und 41,5 g Kaliumjodid in 250 ml Wasser zugetropft. Die dabei entstehende gelbe Emulsion wird am Rotationsverdampfer vom Methanol befreit, mit Salzsäure angesäuert und anschliessend mit Chloroform extrahiert. Zur Abtrennung des 4,6-Dijod-5-indanols wird die getrocknete organische Phase über Kieselgel filtriert, wobei man ein Gemisch von 4-Jod-5-indanol und 6-Jod-5-indanol (Verhältnis ca. 1:2) isoliert, welches als solches weiterverwendet wird.

b) Zu einer Lösung von 15 g Isomerengemisch, bestehend aus 4-Jod-5-indanol und 6-Jod-5-indanol in einem Gemisch aus 75 ml

Eisessig und 7,5 ml Schwefelsäure werden unter Rühren bei Raumtemperatur portionenweise 7 g N-Hydroxymethyl-chloracetamid eingetragen und die Lösung anschliessend 2 1/2 Stunden nachgerührt. Die dunkle Lösung wird auf Eiswasser gegossen, die wässrige Phase mit Aether extrahiert und nach Trocknung der organischen Phase das Lösungsmittel am Rotationsverdampfer entfernt. Dabei resultiert das Isomerengemisch aus 4-Jod-6-[(N-chloracetyl)-aminomethyl]-5-indanol und 6-Jod-4-[(N-chloracetyl)-aminomethyl]-5-indanol als dunkles Oel, welches als solches weiterverwendet wird.

Beispiel 8: Herstellung von 10'000 Tabletten mit einem Gehalt von je 50 mg aktiver Substanz:

Bestandteile:

| | |
|---|---|
| 4-Jod-6-aminomethyl-5-indanol-hydrochlorid | 500 g |
| Milchzucker | 1207 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

Verfahren:

Sämtliche festen Bestandteile werden durch ein Sieb von 0,6 mm Maschenweite gesiebt. Der Wirkstoff wird dann mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser zugesetzt. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 9: Tabletten enthaltend 20 mg an aktiver Substanz werden in folgender Zusammensetzung in üblicher Weise hergestellt:

Zusammensetzung:

| | |
|---|---|
| 1,1-Dimethyl-4-jod-6-aminomethyl-5-indanol-hydrochlorid | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

1,1-Dimethyl-4-jod-6-aminomethyl-5-indanol-hydrochlorid wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 10: Kapseln enthaltend 10 mg an aktiver Substanz werden wie folgt auf übliche Weise hergestellt:

Zusammensetzung:

| | |
|---|---|
| 1,1-Dimethyl-4-aminomethyl-6-jod-5-indanol-hydrochlorid | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Patentansprüche

1. Basisch substituierte Phenole der Formel

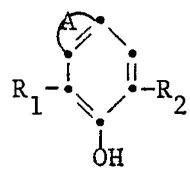

(I),

worin einer der Reste $R_1$ und $R_2$ jeweils für Halogen und der andere für die Gruppe der Formel $-CH_2NH_2$ steht, und A Niederalkylen mit 3 bis 6 Kohlenstoffatomen bedeutet, worin 3 bis 4 Kohlenstoffatome in der unverzweigten Kette stehen.

2. Verbindungen der Formel I, worin einer der Reste $R_1$ und $R_2$ jeweils für Halogen und der andere für die Gruppe der Formel $-CH_2NH_2$ steht, und A Niederalkylen mit 3 bis 6 Kohlenstoffatomen bedeutet, worin 3 Kohlenstoffatome in der unverzweigten Kette stehen.

3. Verbindungen der Formel I, worin einer der Reste $R_1$ und $R_2$ jeweils für Halogen und der andere für die Gruppe der Formel $-CH_2NH_2$ steht und A für 1,1-Dimethyl-1,3-propylen oder 3,3-Dimethyl-2,4-butylen steht.

4. 4-Jod-6-aminomethyl-5-indanol.

5. 4-Aminomethyl-6-jod-5-indanol.

6. 1,1-Dimethyl-4-jod-6-aminomethyl-5-indanol.

7. 1,1-Dimethyl-4-aminomethyl-6-jod-5-indanol.

8. Salze von Verbindungen der Ansprüche 1 bis 7.

9. Pharmazeutisch verwendbare nicht-toxische Säureadditionssalze von Verbindungen der Ansprüche 1 bis 7.

10. Verbindungen der Formel I als pharmakologisch aktive Verbindungen.

11. Verwendung von Verbindungen der Formel I zur Bekämpfung von Oedemen und der Hypertonie.

12. Pharmazeutische Präparate enthaltend eine Verbindung der Ansprüche 1 bis 7 oder 9 zusammen mit einem pharmazeutischen Trägermaterial.

13. Verwendung von pharmazeutischen Präparaten enthaltend eine Verbindung der Ansprüche 1 bis 7 oder 9 als Diuretika und Saluretika.

14. Verfahren zur Herstellung basisch substituierter Phenole der Formel

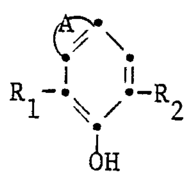

(I),

worin einer der Reste $R_1$ und $R_2$ jeweils für Halogen und der andere für die Gruppe der Formel $-CH_2NH_2$ steht, und A Niederalkylen mit 3 bis 6 Kohlenstoffatomen bedeutet, wovon 3 bis 4 Kohlenstoffatome in der unverzweigten Kette stehen, dadurch gekennzeichnet, dass man

a) in einer Verbindung der Formel

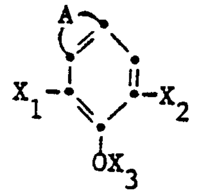

(II),

worin einer der Reste $X_1$ und $X_2$ jeweils Halogen, und der andere die Gruppe der Formel $-CH_2NH_2$ Ia oder einen in die Gruppe der Formel Ia überführbaren Rest darstellen, $X_3$ für Wasserstoff steht, oder $X_1$ und $X_3$ zusammen bzw. $X_2$ und $X_3$ zusammen einen unter Bildung der Hydroxygruppe und der Gruppe Ia abspaltbaren Rest darstellen und A die unter der Formel I angegebene Bedeutung hat, mit der Massgabe, dass eines der Symbole $X_1$ und $X_2$ für einen in die Gruppe der Formel Ia überführbaren Rest steht, und das andere Halogen bedeutet, oder in einem Salz davon, einen in eine Gruppe der Formel Ia überführbaren Rest $X_1$ oder $X_2$ in die Gruppe der Formel Ia überführt, oder die Reste $X_1$ und $OX_3$ bzw. $X_2$ und $OX_3$ gleichzeitig in die Gruppe Ia bzw. die Hydroxygruppe umwandelt , oder

b) in einer Verbindung der Formel

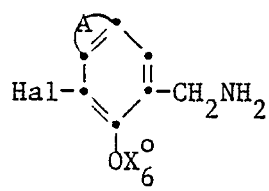

(XIII),

worin $X_6^o$ einen abspaltbaren, durch Wasserstoff ersetzbaren Rest bedeutet, oder in einem Salz davon, den Rest $X_6^o$ abspaltet und durch Wasserstoff ersetzt, oder

c) eine Verbindung der Formel

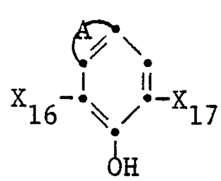

(XVII),

worin einer der Reste $X_{16}$ und $X_{17}$ die Gruppe der Formel Ia und der andere Wasserstoff bedeutet, halogeniert, oder

d) in einer Verbindung der Formel

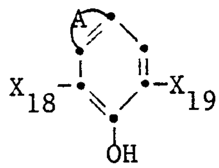

(XIX),

worin einer der Reste $X_{18}$ und $X_{19}$ Halogen bedeutet, und der andere für die Gruppe der Formel $-CH_2X_{20}$ steht, worin $X_{20}$ eine reaktionsfähige veresterte Hydroxygruppe bedeutet, die Gruppe $X_{20}$ gegen die primäre Aminogruppe austauscht, oder

e) in einer Verbindung der Formel

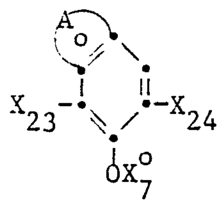

(XXII),

worin $A_o$ einen Niederalkenylenrest bedeutet, oder die Gruppe der Formel -A'-C(=O)- (Ib) darstellt, worin A' für den eine Methylengruppe weniger aufweisenden Rest A steht, einer der Reste $X_{23}$ und $X_{24}$ jeweils Halogen und der andere die Gruppe der Formel Ia oder eine mittels Reduktion in die Gruppe der Formel Ia überführbaren Rest darstellen, und $X_7^o$ Wasserstoff oder einen mittels Hydrogenolyse abspaltbaren und durch Wasserstoff ersetzbaren Rest bedeutet, oder in einem Salz davon, den Rest $A_o$ zur Gruppe A reduziert, und zugleich eine gegebenenfalls vorhandene, mittels Reduktion in die Gruppe der Formel Ia überführbare Gruppe $X_{23}$ oder $X_{24}$ zur Gruppe der Formel Ia reduziert, und/oder zugleich einen gegebenenfalls vorhandenen, mittels Hydrogenolyse abspaltbaren und durch Wasserstoff ersetzbaren Rest $X_7^o$ abspaltet und durch Wasserstoff ersetzt, und wenn erwünscht, eine erhaltene freie Verbindung in ein Salz oder ein erhaltenes Salz in eine freie Verbindung überführt, oder ein erhaltenes Racemat in die optischen Antipoden auftrennt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0020301

Nummer der Anmeldung

EP 80 81 0169

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 709 721 (E.M. SCHULTZ)<br>* Spalte 1, Zeilen 23-70; Tabel 1, Nr. 13,14 *<br>-- | 1 |
| P | DE - A - 2 855 064 (ONO PHARMACEUTICAL CO., LTD.)<br>* Beispiele 12,21; Ansprüche *<br>& FR - A - 2 413 358<br>& GB - A - 2 013 655<br>---- | 1-12, 14 |

**KLASSIFIKATION DER ANMELDUNG (Int.Cl.³)**

C 07 C 91/30
A 61 K 31/135//
C 07 C 65/26
65/36
103/29
103/38
45/46
49/84
43/23
41/26
43/225
41/22
93/14
29/16
29/62

**RECHERCHIERTE SACHGEBIETE (Int. Cl.³)**

C 07 C 91/30
A 61 K 31/135

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-12,14

Unvollständig recherchierte Patentansprüche:

Nicht recherchierte Patentansprüche: 13 Verfahren zur chirurgischen oder therapeu-

Grund für die Beschränkung der Recherche: tischen Behandlung des menslichen oder tierischen Körpers. (Siehe Art. 52(4) des Europäischen Patentübereinkommens).

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

| Recherchenort | Abschlußdatum der Recherche | Prüfer |
|---|---|---|
| Den Haag | 13-08-1980 | PAUWELS |

EPA Form 1505.1 06.78